# EUROPEAN PATENT APPLICATION

(11) **EP 1 336 846 A1**
(43) Date of publication of application: **20.08.2003**
(21) Application number: 02003352.8
(22) Date of filing: 13.02.2002
(51) Int. Cl.: G01N 33/50, C12N 15/87

(54) **Method for generating recombinant human platelets for identifying therapeutic target proteins**

(71) Applicant: Procorde GmbH, 82152 Martinsried (DE)
(72) Inventor: Peluso, Mario, 82377 Penzberg (DE); Ungerer, Martin, PD Dr., 80799 München (DE); Gawaz, Meinrad, Prof. Dr., 80805 München (DE); Massberg, Steffen, Dr., 80339 München (DE); Laugwitz, Karl Ludwig, 82152 Martinsried (DE); Gillitzer, Angelika, 82152 Martinsried (DE)
(74) Representative: Wächtershäuser, Günter, Prof. Dr.

(57) **Abstract**

A composition is provided comprising platelets having a combination of the following modifications:
(a) a modification of the protein constituents of the platelets which is obtained or obtainable by genetic modification of platelet precursor cells;
(b) incorporation of a detectable label into said platelet;
whereby the functions of modification (a) and (b) are mutually independent.

Further, methods of determining platelet functions, notably aggregation and adhesion to endothelial cells are provided. Further, a novel method of preparing transgenic or modified platelets is provided.

## Description

### Field of the Invention

The present invention relates to modified or transgenic platelets and to the use of such platelets as a probe of platelet function, preferably in a blood vessel. Further, the invention relates to a method of determining platelet adhesion to a vessel wall and to a method of determining platelet aggregation. Moreover, the invention relates to a highly efficient method of preparing transgenic platelets. The invention enables the identification of novel drug targets, notably for the treatment of vascular diseases, in particular atherosclerosis as well as venous and arterial thrombosis. Moreover, the present invention provides targets useful in the screeing for novel drug candidates. Moreover, the novel drug candidates may be validated *in vivo* or *in vitro* by using the transgenic platelets of the invention.

### Background

Coronary heart disease and stroke currently account for almost 50% of all deaths in the United States. The interaction of platelets with the vessel wall plays a central role in the development of these and similar diseases by inducing arterial or venous thrombosis with subsequent disturbance of macro- and microcirculation. During adhesion/aggregation platelets shed active mediators, which have a decisive impact on the function of the vessel wall, on other blood cells and on the surrounding tissues. These are pivotal steps during the initiation and further propagation of a vessel thrombus. A prolonged presence of these mediators in blood vessels triggers arteriosclerosis of their walls. Thereby, platelets play a key role not only in the development of coronary heart disease or vessel disease *per se*, but also for the development of arteriosclerotic complications.

Initially, circulating platelets are recruited to the vessel wall by their interaction with the endothelium. They trigger the formation of aggregates, which hinder or completely interrupt arterial blood flow and therefore disturb the nutritive perfusion of the dependent tissues. A thrombotic vessel closure leads to the clinical syndromes of acute myocardial infarction, ischemic stroke or infarction of other organs which are all characterized by the formation of ischemic tissue necrosis. These events can occur acutely, but also in a chronically repetitive way and contribute to tissue ischemia with progressive tissue damage (e.g. in chronically ischemic heart disease with the resulting ischemic heart failure as a disease end point). Beside the acute or chronic impact on arterial blood flow, an increased interaction of platelets with the endothelium also leads to a chronic-inflammatory, proliferative tissue response induced by the release of active mediators. Thereby, the interaction between platelets and the endothelium markedly contributes to the development of the morphological syndrome of arteriosclerosis. Platelets constitute a pivotal and early active component in the pathophysiology of vascular disease and therefore represent an attractive pharmacological target.

Platelets play a pivotal role in a variety of diseases, such as heart disease, stroke, renal infarction or peripheral artery disease and other vascular diseases and in various hematologic disorders. Existing antiplatelet compounds inhibit aggregation and some platelet signaling pathways, but they do not influence other platelet functions such as their shape change during activation or the secretion of vasoactive agents. Therefore, novel drug targets should be identified to improve the treatment of the above-mentioned diseases.

Currently existing antiplatelet compounds have markedly improved the prevention and therapy of acute thrombotic processes. Especially, glycoprotein IIb/IIIa antagonists are efficient but must be given intravenously and have to be strictly controlled. Moreover, vessel damage caused by the release of mediators from platelets cannot be influenced by existing anti-platelet strategies.

An important prerequisite for the development of attractive novel pharmacologic compounds is a more detailed understanding of platelet-endothelial interaction and of their subsequent activation and secretion. Especially, an understanding of the early phases of platelet activation would allow to find new approaches for specific and efficient intervention in platelet-dependent pathological processes, and thereby, a selective inhibition of the subpopulation of *activated* platelets (existing therapies non-specifically inhibit all platelets in the body).

Such innovative antiplatelet compounds would offer the potential of a thrombolysis with markedly reduced side effects, or of highly effective therapies against chronically arteriosclerotic disease and venous disease (such as venous thrombosis or pulmonary embolism), as much as for an effective prophylaxis in high risk vascular patients. The development of such novel antiplatelet therapies by using promising platelet targets intimately depends on a more detailed molecular understanding and characterization of biochemical and signal transduction pathways in platelets. The technology described on the following pages allows to characterize the above-mentioned mechanisms more exactly and to influence potential pharmacological targets selectively by using somatic gene transfer into megakaryocytes. Platelets themselves cannot be genetically modified, because - as nucleus-less cells - they do not possess a DNA-protein synthesis apparatus.

### Current methods to identify novel target proteins in platelets

Out of the approximately 30 000 different proteins in every cell only a small part is functionally relevant at any given moment, depending on the respective pathophysiological condition. Therefore, the information offered by genomic investigation is rather static, whereas all cells - among them platelets - are rather dynamic units, in which proteins are regulated by disease conditions. Especially in the case of platelets, the development of novel therapeutics rather depends on a detailed understanding of protein function ("functional proteomics") in pathophysiological conditions than on genomic investigations. Since the blueprint for all existing proteins, i.e. the genomic DNA sequence, has been identified by the human genome project, the challenge now is to define disease-relevant theraputic targets on the protein level.

One of the most straightforward approaches to study new drug target proteins is to overexpress them in the desired target tissues and cells, or to inhibit them by dominant negative mutant overexpression. Also target proteins in platelets could be investigated in a similar manner, and the effect of these interventions on platelet physiology and on disease endpoints could be studied. So far, however, platelets cannot be used for gene transfer directly, as they do not express foreign transgenes due to the absence of a nucleus in these cells. In particular, the present invention therefore relates to a method which allows for the study of the relevance of protein targets in human platelets ex vivo and in vivo by overexpressing these proteins. Thereby, the invention relates to methods of identifying compounds capable of treating the above-mentioned diseases (acute vessel diseases such as myocardial infarction, stroke, peripheral arterial disease, renal infarction and others; chronic atherosclerosis; venous disease and pulmonary embolism; or hematologic disorders).

Current research projects rely on the following techniques:
1. Quantitive or qualitative investigation of single platelet proteins or enzymatic activities in patients or different animal models of disease.
2. Proteomics: non-directed "screening" of differential protein expression in diseased or healthy platelets; analysis by mass spectrometry or microsequencing.
3. Transgenic mice: offer the potential to overexpress specific genes in target tissues, among them in megakaryocytes. Their generation and cross-breeding with disease models, however, takes several months to years.

These methods generate a variety of hardly comparable single results (1) or are very time consuming (2, 3). Due to the vast amount of data generated by methods (2) or (3), their functional relevance often remains unclear. This problem still impedes the fast and efficient identification of novel compounds directed against platelet-induced disease.

### Current methods of generating recombinant platelets

The use of recombinant platelets has only been investigated for the purpose of hematological research. The group of Choi and Hunt had shown that platelets can be generated from megakaryocytes *in vitro*, and that they have some characteristic morphological features of mature platelets (Choi ES, Nichol JL, Hokom MM, Hornkohl AC, Hunt P. Platelets generated in vitro from proplatelet-displaying human megakaryocytes are functional. Blood 1995; 85:402-413). This group has not tested any expression of foreign transgenes. For hematological purposes, such an overexpression of transgenes in megakaryocytes was proposed by others either by retroviral gene transfer (Burstein SA, Dubart A, Norol F, Debili N, Friese P, Downs T, Yu X, Kincade PW, Villeval JL, Vainchenker W. Expression of a foreign protein in human megakaryocytes and platelets by retrovirally mediated gene transfer. Exp Hematology 1999; 27:110-116; Wilcox DA, Olsen JC, Ishizawa L, Bray PF, French DL, Steeber DA, Bell WR, Griffith M, White GC III. Megakaryocyte-targeted synthesis of the integrin β3-subunit results in the phenotypic correction of Glanzmann thrombasthenia. Blood 2000; 95:3645-3652) or adenoviral gene transfer (Faraday N, Rade JJ, Johns DC, Khetawat G, Noga SJ, DiPersio JF, Jin Y, Nichol JL, Haug JS, Bray PF. Ex vivo cultured megakaryocytes express functional glycoprotein IIb-IIIa receptors and are capable of adenovirus-mediated transgene pression. Blood 1999; 94:4084-4092). The use of tissue-specific promotors has been shown (Wilcox DA, Olsen JC, Ishizawa L, Griffith M, White GC III Integrin alpha IIb promotor-targeted expression of gene products in megakaryocytes derived from retrovirus-transduced human hematopoietic cells. Proc Natl Acad Sci USA 1999; 96: 9654-9659).

In all studies published to date, however, the use of gene transfer into the progenitor cells and subsequent use of recombinant platelets for studying vascular biology, and thereby investigating drug targets relevant for heart and circulatory diseases ex vivo and preferably in vivo has never been mentioned. Further, such applications of recombinant platelets have been out of reach so far due to insufficient platelet numbers achieved by cytokine-induced shedding of functional platelets.

Currently available technologies of finding drug targets in platelets rely on the study of peripheral human or animal platelets both in clinical and in experimental studies. This approach offers the advantage of measuring physiological parameters directly, for instance in response to a therapy with existing anti-platelet drugs. However, this approach very often does not allow to test specific molecular effects of novel signalling pathways nor to identify novel drug targets. This is partly due to the fact that existing compounds such as acetylsalicylic acid have a simultaneous impact on several biological pathways and target proteins in platelets. Therefore, a method to relate molecular mechanisms to the pathology of vascular and other diseases did not exist before.

It is therefore a problem of the invention to provide novel methods of identifying new drug targets preferably in platelets, especially for treating heart and vascular diseases.

It is another problem of the invention to provide new methods of determining platelet functions, notably in vivo.

It is another problem of the invention to provide new modified platelets.

It is another problem of the invention to provide new methods of generating modified platelets in larger amounts than possible with methods of the prior art.

It is another problem of the invention to provide a method of inducing highly efficient shedding of platelets from megakaryocytes.

### Description of the invention

This invention provides a composition comprising platelets having a combination of the following modifications:
(a) a modification of the protein constituents of the platelets which is obtained or obtainable by genetic modification of platelet precursor cells;
(b) incorporation of a detectable label into said platelet;
whereby the functions of modification (a) and (b) are mutually independent.

The present inventors have found that the composition of the invention may be used as a probe for platelet function. Notably, said composition may be used as a probe for platelet function in a model animal, preferably in a blood vessel of a model animal. Therefore, the composition may be used for studying the function of a gene or its gene product (protein) expressed in platelet precursor cells and present in platelets in the context of the vascular system and in the context of vascular diseases. This invention allows for the first time to investigate and to determine the function of proteins in platelets in vivo. The identification of functions of platelet proteins notably in vivo is of utmost importance for identifying new drug targets for treating vascular diseases like atherosclerosis, thrombosis, heart infarction, stroke, bleeding diseases etc.

Genetically modified or recombinant platelets are already known in the art. However, these platelets were created for academic purposes and the methods used for creating these platelets were inefficient. Recombinant platelets have never before been used to screen for drug targets for treating diseases involving platelets. For the first time, this invention makes available sufficient amounts of ex vivo generated modified platelets for such studies.

Platelet functions to be probed by said composition of the invention may be any function which involves platelets or platelet components, whereby said functions may involve further factors or components of the vascular system like endothelial cells or factors of the blood coagulation cascade (e.g. fibrinogen). Said platelet functions are preferably functions platelets have in vivo. Examples of platelet functions to be probed are platelet activation, platelet aggregation, platelet interaction with or adhesion to endothelium, secretion of factors from platelet granula, activation of endothelial cells by platelets or by platelet-secreted factors etc. Most preferred functions to be probed involve platelet aggregation and platelet adhesion to endothelial cells.

The invention discloses methods of determining platelet functions in vivo using said composition comprising platelets. Specifically, the invention discloses a method of determining platelet adhesion to a vessel wall, whereby the method comprises the steps of
(a) exposing the interior wall of a blood vessel under predetermined conditions with said composition of the invention;
(b) determining the adhesion of the detectably labelled platelets to the wall of said blood vessel.

Further, the invention specifically discloses a method of determining platelet aggregation in a blood vessel, whereby the method comprises the steps of
(a) exposing the interior wall of a blood vessel under predetermined conditions with said composition of the invention;
(b) determining the aggregation of the detectably labelled platelets in said blood vessel.

Said methods are preferably carried out in vivo in a blood vessel of a model animal. Said blood vessel may be a healthy blood vessel of a healthy model animal. Said blood vessel may also be a blood vessel affected by a vascular injury or a vascular disease. Preferably, said blood vessel is an ischemic blood vessel or an atherosclerotic blood vessel. Said blood vessel may be an artery or a vein. An artery is preferred. More preferred is a carotid artery or an intestinal capillary bed. However, said blood vessel may be any blood vessel which is accessible experimentally for observing platelet aggregation or adhesion of platelets to a vessel wall, notably in vivo.

Said model animal should be related to humans with respect to their vascular system. Preferably, said model animal is a mammal. Further, said model animal should be suitable for in vivo experimentation. Preferred examples of such animals are mice, rats, rabbits etc. Most preferred are mice.

Said model animal may be genetically modified, i.e. it may be transgenic or it may have been subjected to gene therapy. Said genetic modification may be a deletion or a knock-out of a natural gene or it may be (over)expression of a native or mutated gene. Preferably, said genetically modified animal is a transgenic mouse, e.g. an ApoE-deficient mouse.

Further, said model animal may be a disease model in order to carry out said methods in a disease model blood vessel. Preferably, said model animal is a model for a vascular injury or a vascular disease like thrombosis, atherosclerosis, ischemia/reperfusion, myocardial infarction, acute coronary syndrome etc. Said disease model may be created by way of genetic modification or by way of subjecting an animal to certain conditions or physical treatment. Further, a disease model in a model animal may be created by breeding methods. Such disease models and methods of their creation are known in the art. An example of a physical treatment producing vascular injury is given in example 14. An example of a disease model created by genetic modification is said ApoE-deficient mouse mentioned above.

Said methods of determining platelet functions in a model animal preferably comprise anesthetising and sacrificing said model animal.

Said methods of determining platelet functions in a model animal may comprise administration of said composition of the invention into the vascular system of said model animal. Preferably, said composition is administered intravenously making use of a catheter. Platelet function, notably platelet aggregation and/or adhesion of platelets to endothelium, may be visualised by intravital videofluorescence microscopy in a blood vessel by way of said detectable, preferably fluorescent, label of the platelets of the invention. A function of the administered platelets of the invention may be compared to a function of platelets carrying said detectable label but no modification of the protein constituents of the platelets. Alternatively, a function of the platelets of the invention in a disease model blood vessel may be compared to a reference blood vessel not affected by said disease. Further, platelet functions may be compared in a model animal of any state or medical or environmental condition like age, sex, drug medication, nutrition, exposure to cigarette smoke etc. with a reference model animal.

Once a drug target protein has been identified according to the invention, drug candidates may be tested using analogous methods. The invention also discloses an assay for screening for drug candidates, wherein a drug target identified using a method for determining platelet function according to the invention is used. Further, an assay for testing new drug candidates by using the composition of the invention is disclosed.

Said composition of the invention may further be used for determining protein function in platelets in vitro. A preferred platelet function to be studied in vitro is platelet aggregation or secretion of active mediators from platelets (cf. examples 11 and 12). Further, flow cytometry may be used to determine the amounts of proteins present in said platelets, preferably on their surface, in response to a modification of the protein constituents (cf. example 9).

The platelets of the composition of the invention have a modification of their protein constituents which is obtained or obtainable by genetic modification of platelet precursor cells. Said precursor cells are preferably megakaryocytes. However, precursor cells of megakaryocytes, notably CD34⁺ cells, may also be genetically modified to obtain modified megakaryocytes and platelets therefrom.

Protein constituents of the platelets refers to the proteome of the said platelets, i.e. all types of proteins which may be present in platelets including their abundance. Said proteome may be that of normal healthy platelets or that of platelets affected by a genetic disorder.

Said modification of the protein constituents of the platelets is obtained or obtainable by genetic modification of platelet precursor cells. Said modification may affect a single or several proteins of platelets. Preferably, said modification affects one protein. Said modification of the protein constituents may relate to (i) the absence or inhibition of one or more proteins; (ii) the overexpression of one or more proteins; and/or (iii) to the presence of a foreign protein capable of modifying an existing protein.

For the pupose of the invention, "absence of a protein" also comprises reduction of the amount of said protein normally present in platelets. Absence of one or more proteins in platelet precursor cells may be achieved by genetic engineering according to known methods. For example, the gene coding for said protein may be deleted fully or partly or may be replaced by a non-functional (e.g. mutated or truncated) form of said protein. Said genetic engineering may make use of homologous recombination. Further, expression of said protein may be suppressed by way of an anti-sense or ribozyme mechanism. Inhibition of a protein may be achieved by introducing a mutation reducing a functionality of said protein or by dominant negative inhibition by expressing a non-functional form of said protein (see below).

Methods of overexpressing one or more proteins in animal cells and in particular in platelet precursor cells are known in the art (cf. examples 7 and 8). Viral vectors like adenovirus-based vectors or retrovirus-based vectors are frequently used for this purpose. The protein to be expressed may be any native mammalian protein or a foreign protein. Preferably, said protein to be (over)expressed is a mammalian protein that is expressed in platelets either under normal, healthy conditions or under the condition of a disease. Said protein is preferably native to humans or native to said model animal. Said foreign protein may be a protein that does not exist in humans e.g. a microbial (bacterial, fungal) gene. Said foreign protein may alter the amount or functionality of a native protein e.g. by cleaving said native protein. Preferably, said cleavage is specific for a particular native protein under investigation.

As a further modification, said platelets of said composition have incorporated a detectable label. Said detectable label may be present internally in said platelets or it may be attached to the outer membrane of said platelets. Said detectable label may be a fluorescent dye or a fluorescent protein. Numerous such dyes and proteins are commonly used in cell biology/microscopy. Among fluorescent dyes, fluorescein, phycoerythrine, and rhodamine may be mentioned. Labeling of biological objects like macromolecules (e.g. antibodies) or cells with such dyes is widely used in cell biology/fluorescence microscopy. Among fluorescent proteins, green fluorescent protein (GFP) and its blue, yellow, orange and red derivatives and relatives of various emission maxima may be mentioned (e.g. those discussed in Nature Biotechnology (2002) 20, 28-29 and references cited therein). Such a fluorescent protein may be expressed in platelet precursor cells according to known methods such that they are present in platelets. Said detectable label may be used for visualising said platelets when determining the function of a modification of the protein constituents of platelets.

The functions of the modification of the protein constituents of the platelets (a) and the modification by incorporation of a detectable label (b) are mutually independent. Specifically, the modification (a) provides a functional platelet wherein the modification of the protein constituents shows the same function of the in the presence or absence of the specific modification (b). In particular, the presence of modification (b) does not interfer with the function of modification (a) to such an extend that the function of modification (a) is eliminated, significantly changed or replaced by another function such that the function of modification (a) observed in the absence of modification (b) cannot be used for the purpose of the invention. The functions of the modifications (a) and (b) are mutually dependent, e.g. in case the detectable label impairs the function of a surface-expressed platelet protein. Specifically, the invention does not relate to the case when the label attaches to the protein modification (a) via an antibody, since the antibody then prevents interaction of said protein with other interaction partners.

If the detectable label is a protein, it may be expressed in platelet precursor cells from a different expression cassette than the protein responsible for said modification (a). However, the same promoter may be used in these expression cassettes. Alternatively, the protein which is the detectable label and a protein which is responsible for said modification of the protein constituents may also be expressed as a fusion protein as long as the functions of said proteins are mutually independent. Whether the functions of the proteins making up a fusion protein are mutually independent may be tested by comparing said functions with the case wherein these proteins are expressed separately (not as a fusion protein). A negligible influence of one modification of said platelets on the other modification may, however, be tolerated.

This invention further discloses a method of preparing platelets for said composition of the invention, wherein the method comprises the following steps:
(a) providing hematopoietic progenitor cells;
(b) generating megakaryocytes based on the hematopoietic progenitor cells of step (a);
(c) selecting a gene of interest from a gene library;
(d) transforming or transfecting the megakaryocytes with a vector containing the functional gene of interest of step (c);
(e) inducing shedding of platelets from said transformed or transfected megakaryocytes of step (d);
(f) isolating platelets containing expression product of the gene of interest and optionally a detectable marker; and
(g) optionally incorporating a detectable label into the platelets obtained in step (f).

Hematopoietic progenitor cells may be prepared from peripheral blood e.g. as described in example 2 or from bone marrow as described in example 5. Said progenitor cells may be prepared from humans or from said model animal, e.g. from an animal species as used for the above methods of determining platelet adhesion or platelet aggregation. Hematopoietic progenitor cells may be enriched according to methods known in the art (cf. examples 3 and 4). Megakaryocytes may then be generated based on the hematopoietic progenitor cells of step (a) (cf. example 6).

In the next step of said method of preparing platelets, a gene of interest is selected from a gene library. Said gene may be selected based on any criteria, e.g. based on information already known in the art.

Subsequently, the megakaryocytes are transformed or transfected with a vector containing the gene of interest selected in step (c) for achieving (over)expression of said gene of interest. Viral vectors like adenovirus-based vectors or retrovirus-based vectors are frequently used for this purpose (cf. examples 7 and 8).

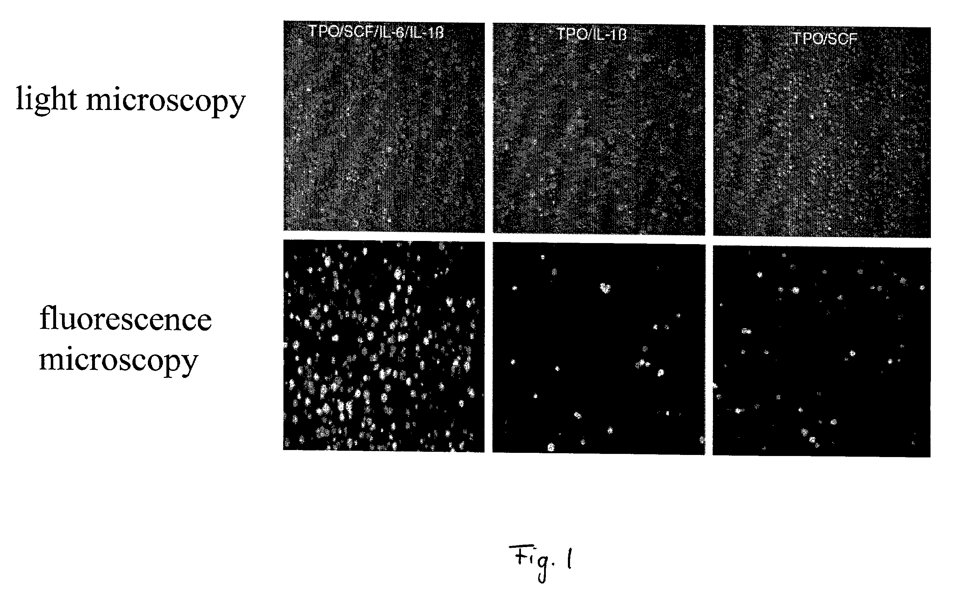

After extensive investigations, the inventors found a unique cytokine cocktail for inducing shedding of platelets from megakaryocytes (step (e)). This cytokine cocktail comprises of thrombopoietin, interleukin-1β, stem cell factor and interleukin-6. These cytokines are preferably used in the following concentration ranges: thrombopoietin (TPO; 10±5 ng/µl), interleukin-1β (IL-1β; 10±5 ng/µl); stem cell factor (SCF; 50±20 ng/µL) and interleukin-6 (IL-6; 10±5 ng/µL). In contrast to previous publications, this unique cytokine cocktail allows for optimized generation of modified platelets, as tested by directly comparing this protocol to conventional ones proposed in the existing relevant literature (Fig. 1): In Burstein SA, Dubart A, Norol F, Debili N, Friese P, Downs T, Yu X, Kincade PW, Villeval JL, Vainchenker W. Expression of a foreign protein in human megakaryocytes and platelets by retrovirally mediated gene transfer. Exp Hematology 1999; 27:110-116, the combination of SCF+TPO or a 6-factor combination of SCF, IL-3, Flt 3 ligand, IL-6, GM-CSF and TPO were used. In Norol F, Vitrat N, Cramer E, Guichard J, Burstein S, Vainchenker W, Debili N: Effects of cytokines on platelet production from blood and marrow CD 34⁺ cells. Blood 1998; 91: 830-443, different cytokine protocols were compared (which did not include the one we used) and TPO (=MGDF) + SCF was identified as the best combination in their hands. Comparing identical concentrations of cytokines on the same preparations in parallel, the new combination of TPO+SCF+IL-6+IL-1β resulted in 8.5±2 x10⁴ modified platelets per 10⁵ megakaryocyte-differentiated stem cells per single harvest, whereas the combination of TPO + SCF only yielded 1.24±2 x10⁴ modified platelets per 10⁵ stem cells, and TPO and IL-1β 0.78±0.5 x10⁴ modified platelets per 10⁵ stem cells (p<0.05 new protocol against conventional ones by ANOVA; all measurements done by FACS).

Platelets containing expression products of the gene of interest are then isolated, preferably by centrifugation.

In the next step, said detectable label may be incorporated into the platelets obtained in the previous step. Types of detectable labels and methods of incorporation into platelets are discussed above and are also known in the art.

The modified platelets of the invention can be used for the measurement of different physiological parameters which proved the excellent comparability of the modified platelets of the invention with freshly isolated human platelets. To clarify the pathophysiologic role of specific intracellular proteins during platelet activation, modified platelets can be investigated by fluorescence-activated cell sorting (FACS) with specific antibodies which show alterations in relevant surface proteins by novel target proteins or their inhibition. The co-expression of e.g. green fluorescent protein (GFP) with a target protein allows to control the presence of the target protein in individual platelets. This approach allows to study the targets protein's effects e.g. on the expression and/or activation of fibrinogen receptors (glycoprotein IIb-IIIa), on the composition of storage vesicles and granules, the externalisation of internal proteins such as CD62P or CD40 ligand, on the degranulation of active compounds (VEGF, PDGF, β-thrombogloblin, platelet factor 4 and others). The aggregation of modified platelets could be investigated directly e.g. by fluorescence microscopy or by luminometric aggregometry, which also allowed to measure ATP release from dense granules. The effect of novel target proteins on these physiologic parameters can then be studied.

### Effect of modified platelets on human endothelial cells

The *ex vivo* generated modified human platelets may be used for different experiments to characterize the effects of platelet-released mediators and platelet adhesion proteins on the function of human endothelial cells e.g. by measuring their chemotactic properties (e.g., release of monocyte chemoattractant protein-1), or their adhesive (e.g. expression of ICAM-1 and vitronectin receptors) or fibrinolytic potential (e.g. matrix metalloproteinases, uPAR) after incubation with activated modified platelets. Central biochemical functions of endothelial cells and important signal cascades can then be characterized e.g. by protein chemistry or FACS. Platelet-induced alterations of endothelial *ex vivo* adhesivity for monocytes, which play an important role in atherogenesis, may be investigated in parallel in cell adhesion assays (incubation of platelet-stimulated HUVECs with monocytes).

### Preparation of mice for measurement of novel target platelets and measurement in vivo

A major advantage of the invention is that it allows to clarify the functional significance of specific target proteins or signal transduction cascades for both platelet-endothelial cell interactions and platelet function *in vivo*. Importantly, this allows to define the role of specific platelet target proteins in pathophysiological settings including atherosclerosis and atherosclerotic tissue remodeling in animal models of disease (e.g. ApoE-deficient mice, cholesterol-fed rabbits) as well as ischemia-reperfusion.

Modified platelets can be used after intravenous administration to directly visualize and quantitatively assess their acute effects on arterial circulation/blood flow in animals by means of intravital videofluorescence microscopy. This can yield valuable data e.g. in the carotid artery which is a pivotal vessel in the pathogenesis of stroke, or in the postischemic intestinal capillary bed, which allows to study the role of platelet target proteins in ischemia-reperfusion, a major consequence of the atherosclerotic process. The invention enables a highly sensitive quantitative analysis of alterations of platelet adhesion to the vessel wall. It allows to study platelet adhesion to previously injured endothelium or atherosclerotic endothelium of Apo E^{-/-}mice. Moreover adhesion to ischemic tissue can be studied (arterially and postcapillary), which also allows to indirectly measure secretion from platelets.

### Short description of the figures

- Fig.1: Generation of modified platelets induced by different cytokine cocktails. Starting from identical amounts of stem cells from the same preparation, the generation of modified platelets using the new combination of TPO+SCF+IL-6+IL-1β (left) was compared to that seen with TPO + IL-1β (middle) or TPO + SCF (right) used at identical concentrations (see example 6).

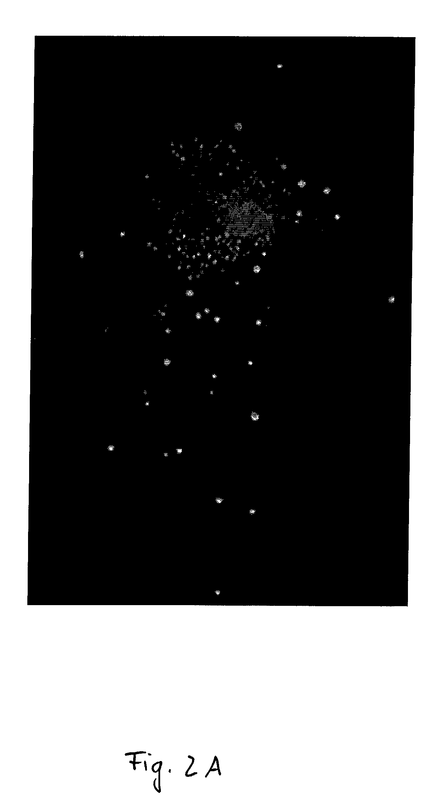

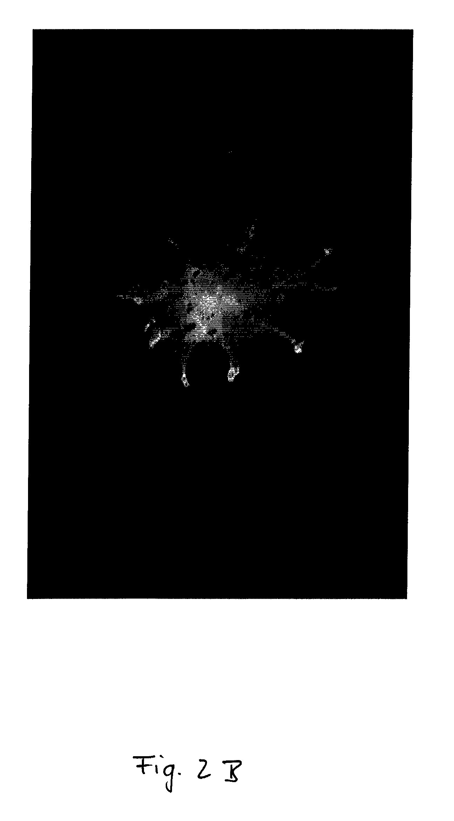

- Fig. 2: A and B show megakaryocytes infected *ex vivo* with modified virus encoding the transgene green fluorescent protein, in a microscopic image under fluorescence light. The megakaryocytes shed modified platelets continuously which can be harvested at different times over several weeks (see example 8).

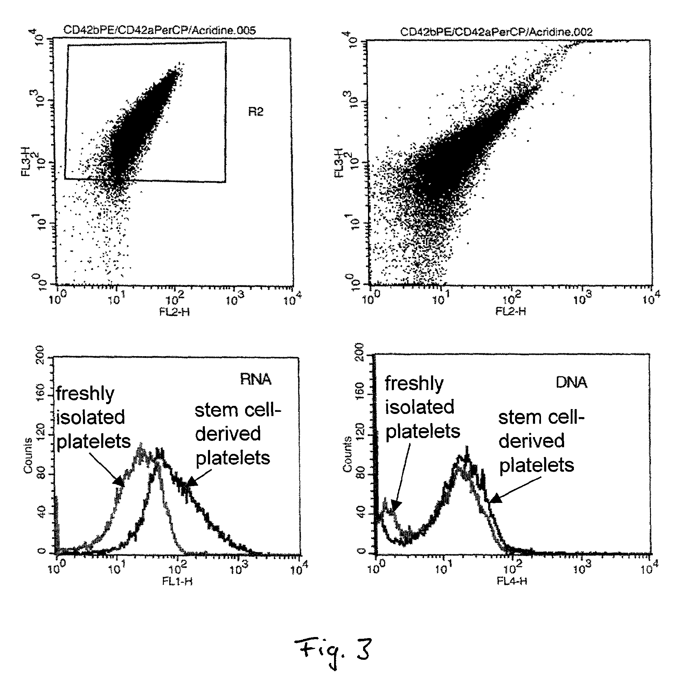

- Fig. 3: FACS measurement of modified platelets showing expression of fibrinogen receptors and its ligand-induced binding site, P-selectin and their activation by thrombin, ADP or the fibrinogen-like peptide RGD. All results compare well to those obtained with freshly isolated platelets (see example 9 for details).

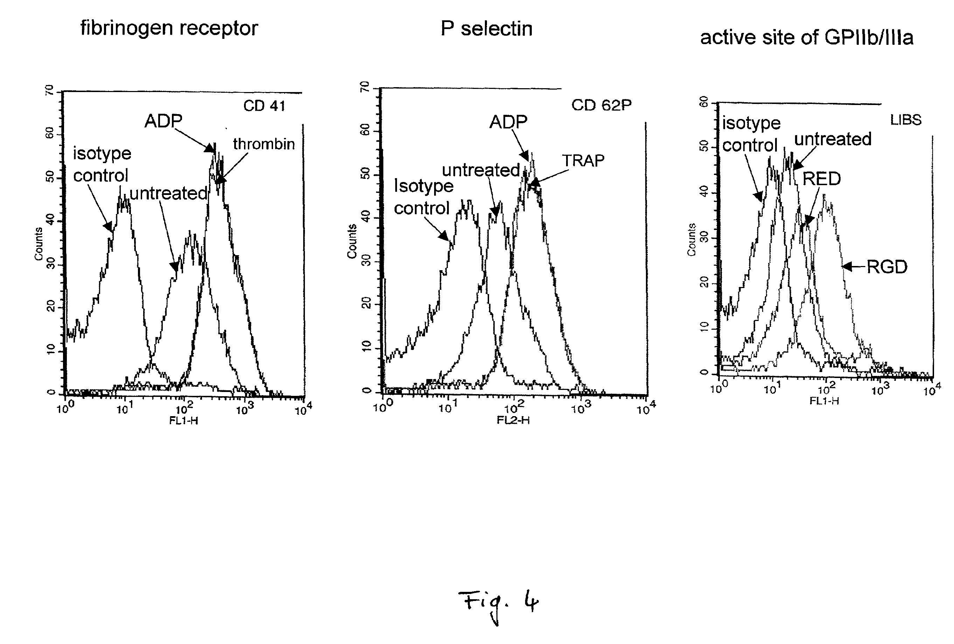

- Fig. 4: Original image of a FACS examination of platelets after acridine orange staining (top). Extinction curves indicate RNA content (left) and DNA content (right). The curves at the bottom indicate freshly isolated platelets and platelets derived from stem cells according to the invention (see example 10 for details).

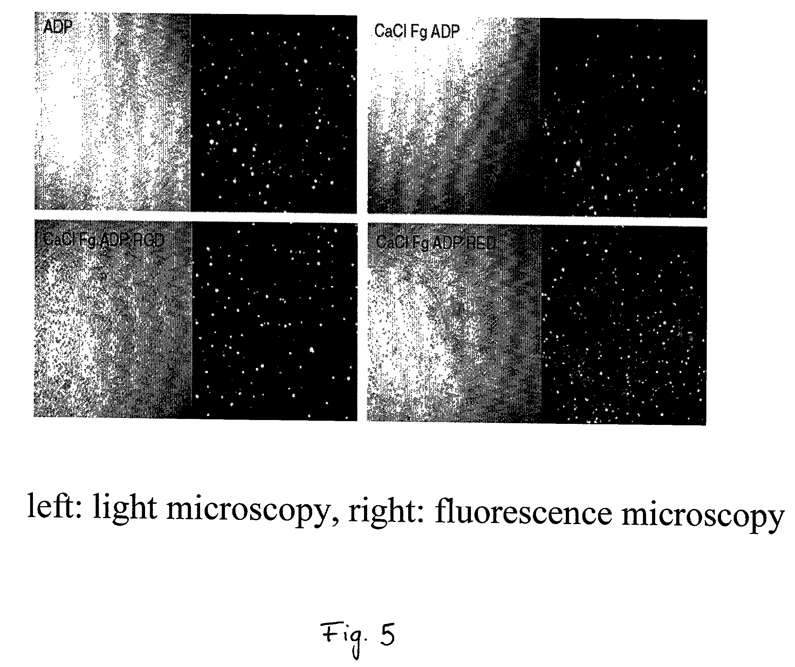

- Fig. 5: Aggregation of modified platelets ex vivo. Aggregation is well comparable to that of freshly isolated platelets. The figure shows that no aggregation occurs in the absence of calcium and fibrinogen (upper left panel), and that aggregation can be specifically blocked by the fibrinogen antagonist peptide RGD (bottom left), but not with the mock peptide RED (bottom right).

### Examples

### Example 1: Protocol for isolation of platelets from fresh blood (sodium citrate-anticoagulated blood)

Freshly drawn blood from human volunteers was transferred into a Falcon tube and spun down for 20 min. at 1080 rpm at 20°C. The supernatant yielded the PRP (platelet-rich-plasma) and was transferred to a new tube and diluted 1 : 4 with wash-buffer (1x PBS (w/o CA²⁺ Mg⁺) with 0.1% sodium azide, pH 7.2 +/- 0.2, filtered through a 0.2 µm filter prior to use and stored at 4°C). The mixture was spun down for 10 min. at 2100 rpm at 20°C. The pellet was resuspended in staining buffer (1x PBS (w/o CA²⁺ Mg⁺) with 0.1% sodium azide and 2% fetal bovine serum) in a volume of 40µl.

### Example 2: Isolation of adult peripheral human stem cells:

The direct CD34 Progenitor Cell Isolation Kit (from Miltenyi Biotech, Bergisch Gladbach, Germany) contains MicroBeads directly conjugated to CD34 antibodies for magnetic labeling of CD34 expressing hematopoietic progenitor cells from peripheral blood, cord blood, bone marrow or apheresis harvest. Hematopoietic progenitor cells, present at a frequency of about 0.05-0.2 % in peripheral blood, 0.1-0.5 % in cord blood and 0.5-3 % in bone marrow, can be rapidly and efficiently enriched to a purity of about 6-98 %.

### Preparation of peripheral blood mononuclear cells:

· Fresh human blood samples were treated with an anticoagulant, e.g. heparin, citrate, ACD-A or phosphate dextrose (CPD). Alternatively, leukocyte-rich buffy coats not older than 8 hours were used.
· The cells were diluted in 1 volume of 1x phosphate buffered saline (PBS; from Sigma, Deisenhofen, Germany) containing 0.6 % CPD-A.
· Cells were carefully layered over 15 ml Percoll® (1.077 density) (Percoll Separating Solution; Biochrom, Berlin, Germany) in a 50 ml conical tube and centrifuged at 1800 rpm for 35 minutes at 20°C in a swinging-bucket rotor (without brake; speed-up = 1, break = 0). Up to 35 ml of diluted cell suspension can be used.
· The upper layer was aspirated leaving the mononuclear cell layer undisturbed at the interphase.
· The interphase cells (lymphocytes and monocytes) were carefully transferred to a new 50 ml conical tube containing 25 ml of PBS containing 0.6 % CPD-A.
· Cells were mixed and centrifuged at 1750 rpm for 10-15 minutes at 20°C (full speed-up and break). The supernatant was completely and carefully removed.
· The cell pellet was resuspended in 10 ml of PBS containing 0.6 % CPD-A and mixed.
· The cells were filtered with a cell strainer to remove fat, and tubes were washed with PBS/CPD-A washing buffer (1xPBS w/o Mg⁺/Ca⁺, 0.6% CPD-A) and centrifuged at 1250 rpm for 20 minutes at 20°C. The supernatant was completely and carefully removed. The cell pellet was then resuspended in a final volume of 2ml staining buffer (500ml 1xPBS w/o Mg⁺/Ca⁺, 0.6% (3ml) CPD-A, 0.5% BSA (35%)) per buffy (PBMC of about 500 ml blood). These cells were then used for magnetic labeling.

### Example 3: Magnetic Labeling of CD34⁺ Progenitor Cells

- 100µl FcR blocking reagent per 108 total cells was added to the cell suspension to inhibit unspecific or Fc receptor-mediated binding of CD34 MicroBeads to non-target cells.
- 500µL of the cell suspension were labelled by adding 100 µl CD34 MicroBeads per 10⁸ total cells, mixed well and incubated for 30 minutes in the refrigerator at 6°-12°C.
- Cells were carefully washed (with cold staining-buffer, ≈25 mL) and resuspended in an appropriate amount of buffer.
- LS + /VS + column: 10-20 ml, max. 1x10⁸ cells per ml.
- Then, magnetic separation was carried out.

### Example 4: Magnetic Separation of <2x10⁹ Mononuclear Cells

To avoid capping of antibodies on the cell surface during labeling all following steps were done rapidly. Cells were constantly kept cold, only cold solutions were used. All buffers were degassed prior to use.
· Positive selection columns (MS + /RS + or LS + /VS + ) were chosen according to the number of total unseparated cells and placed (with a column adapter) in the magnetic field of the MACS separator (Miltenyi Biotech, Bergisch Gladbach, Germany). They were filled and rinsed with buffer (MS +/RS +: 500 µl; LS + /VS +: 3 ml; for details, see "Column and Adapter Data Sheets" of the manufacturer).
· Cells were passed through 30 µm nylon mesh or filter (Miltenyi Biotech; Order No.414-07) to remove clumps; before use filters were wetted with buffer.
· Cells were applied to the column and allowed to pass through the column and washed with buffer (MS + /RS + : 3 x 500 µl; LS + /VS + : 3 x 3 ml).
· The column was removed from the separator, placed on a suitable tube, and buffer was pipetted on top of the column (MS + /RS + : 1 ml; LS + /VS + : 5 ml). Retained cells were eluted using the plunger supplied with the column.
· The magnetic separation step was repeated: the eluted cells were applied to a new prefilled positive selection column (for <10⁷ CD34 + cells: MS + /RS + ; for <10⁸ CD34 + cells: LS + /VS + ), washed, and retained cells were eluted in buffer (MS + /RS + : 500 µl; LS + /VS + : 2.5 ml).

### Example 5: Isolation of stem cells from mouse bone marrow

Bone marrow cells were harvested by flushing the femurs and tibias of mice with 1x PBS (w/o Ca⁺, Mg⁺) with 0.6% CPD-A and 0.5% BSA (MACS-buffer). The cells were washed twice (spun down for 10 min., at 1800 rpm), aspirated through 20- and 25-gauge needles and filtered through Nytex mesh to break clumps and to ensure that mature MK's were destroyed.

The remaining cells were layered over Percoll (15mL). Mononuclear cells were separated by centrifugation for 30 min. at 1800rpm, RT, without break and speeding. Cells were washed in Macs medium (1x PBS (w/o Ca⁺, Mg⁺) with 0.6% CPD-A, 0.5% BSA). For some experiments, cells were stained with SCA1 microbeads antibodies and positive cells were separated in a magnetic field according to the protocols of 4.3 and 4.4. Cells were then plated out in Optimem (Glutamax I) for 2 hours in 6 well-plates. Non-adherent cells were collected and resuspended in specific growth medium. The preceding steps were repeated after over-night culturing to remove adherent subset of populations.

### Example 6: Generation of megakaryocytes

For all following steps, the medium IMDM (from LifeTechnologies-Gibco; Wiesbaden, Germany) was used, to which stable glutamine (Glutamax II; LifeTechnologies-Gibco), 1.5 % bovine serum albumin (BSA; Sigma, Deisenhofen), 300 µg/mL iron-saturated transferrin, 1 mM sodium pyruvate, 1x MEM vitamins (from Life Technologies-Gibco; Wiesbaden, Germany), 0.02 mg/mL L-aspargine, 0.01 mM of the antioxidant monothioglycerol, 1x non essential amino acids (Life Technologies); penicillin/streptomycin 10ng/mL were added to the cells.

After extensive testing (see below) the following human or mouse cytokines (all from R+D systems, Wiesbaden) were used for differentiation: thrombopoietin (TPO; 10 ng/µl), interleukin-1β (IL-1β; 10 ng/µl); stem cell factor (SCF; 50ng/µL) and interleukin-6 (IL-6; 10ng/µL).

Comparing identical concentrations of cytokines on the same preparations in parallel, the new combination of TPO+SCF+IL-6+IL-1β resulted in 8.5±2 x10⁴ modified platelets per 10⁵ megakaryocyte-differentiated stem cells per single harvest (Fig. 1), whereas the combination of TPO + SCF only yielded 1.24±2 x10⁴ modified platelets per 10⁵ stem cells, and TPO and IL-1β 0.78±0.5 x10⁴ modified platelets per 10⁵ stem cells (p<0.05 new protocol against conventional ones by ANOVA; all measurements done by FACS).

Fresh medium was added to all cells every 6-8 days: ∼300-400µL of old medium was discarded from the wells by pipetting from the surface and exchanged by 300-400µL fresh medium (drop by drop). Platelets were harvested by centrifugation (in undiluted medium) at 1600 rpm for 1¼ min. 20°C. The supernatant was transferred into a new tube (leaving ∼1 mL) and diluted 1:2 with 1x PBS w/o Mg⁺,Ca⁺, 0.5% FBS (in platelet wash buffer), then spun down 5min at 1600rpm. Megakaryocytes were replated after the separation of platelets with fresh growth medium in the same well.

### Example 7: Protocol for adenoviral infection of megakaryocytes

Modified (E1/E3-deficient) flag-tagged adenoviruses for novel transgenes were generated that for expressing a transgene and green fluorescence protein (GFP) under control of two independent CMV promoters in a bi-cistronic system (He TC, Zhou S, da Costa LT, Yu J, Kinzler KW, Vogelstein B. A simplified system for generating modified adenoviruses. Proc Natl Acad Sci USA. 1998; 95:2509-14, purchased from Q Biogene, Heidelberg, Germany). As a control, Ad-GFP without further transgene was used. Large virus stocks were prepared and purified by centrifugation via cesium chloride density gradients. Adenoviral titers were determined using plaque titration and GFP expression titration in non-E1-expressing cells.
1) Megakaryocytes were harvested at day 8-11 after isolation using a wide sterile single-use pipette and spun down in a 15mL Falcon-tube at 1650 rpm for 8 min.
2) The supernatant was discarded and the pellet resuspended in Optimem (at 4x10⁵ cells/300µL).
3) Adenovirus was added to each well of a 24 well-plate for infection (multiplicity of infection (MOI) of 400 plaque forming units per cell).
4) Cells were added and mixed by shaking, incubated for 30min at 37°C at 5% CO₂, then
5) spun down at 1000 rpm, 20°C for 10 min.

They were carefully pipetted from the surface with 150µL Optimem, and 600µL growth-medium was added. Typically, transgene expression occurred 48 hours thereafter and persisted for 3 weeks. Measurements could be done repeatedly during this period. Platelets could be harvested continuously.

### Example 8: Protocol for the retroviral infection of megakaryocytes

Modified retrovirus were generated by using the pantropic retroviral expression system sold by Clontech, Heidelberg, Germany (order no. K1063-1). The HEK 293-based packaging cell line GP2-293 was propagated in complete medium according to the manufacturer's instructions. The genes of interest were cloned into a pLEGFP-C1 (Clontech cat. no. 6058-1), so that they were fused to GFP. Alternatively, HA-tagged genes were cloned into pLNCX-2 (Clontech, cat. no 6102-1). To produce infectious virus, the cells were cotransfected with pLNCX (Clontech) and pVSV-G (Clontech) by a calcium phosphate CalPhos transfection kit (Clontech, order no. K2051-1). The virus was harvested up to four times starting 48 hours after transfection and concentrated in a Centricon plus 80 column (Millipore, cat no. UFC5 LTK08) and titrated in NIH3T3 cells (mean titers of 5 x 10⁵ infectious units/ml). For infection, retroviruses were applied for 3 hours at 37°C to differentiating megakaryocytes 7-11 days after isolation (moi of 1-4 virus/cell), using Polybrene reagent (4 µg/ml) from Sigma (Deisenhofen, Germany). Alternatively, a coninfection protocol according to Burstein et al., 1999, was used.

### Example 9: Protocol for platelet staining with antibodies

1) Platelets were harvested by centrifugation at 1800 rpm for 1¼ min at 20°C; the supernatant was diluted 1:1 with platelet wash buffer (1x PBS (w/o CA²⁺ Mg⁺) with 0.1% sodium azide (pH 7.2 +/- 0.2)).
2) They were filtered through a 0.2 µm filter prior to use, stored at 4°C and spun down at 1800 rpm for 5 min.
3) The supernatant was discarded. ≈1mL was left in the tube and washed again with staining buffer (1x PBS (w/o CA²⁺ Mg⁺) with 0.1% sodium azide and 2% fetal bovine serum (FBS ) and stored at 4°C
4) The supernatant was completely discarded.
5) The pellet was resuspended in platelet-staining-buffer (40 µL/setting).
6) 20 µL of the activators thrombin receptor activating peptide (TRAP; 25 µM), ADP (5 or 20 µM) or thrombin (2 U/ml) were added. 35µL of the fibrinogen receptor binding peptide RGD or its negative control, RED, were added where indicated.
7) The mixture was incubated for 10 min. at room temperature in the dark.
8) Antibodys were added directly (in a volume of 7µL each except for LIBS-FITC at 3.5 µL) and incubated for 20-30 min. at room temperature in the dark.
9) 500-600µL platelet staining buffer was added. Then, the mixture was spun down for 5 min. at 1800 rpm (in a tabletop centrifuge). The supernatant was discarded, 300-400µL staining-buffer were added. FACS measurements were done immediately.

For the following studies, antibodies against the following antigens were used:
- CD 41: complexed fibrinogen receptor/glycoprotein IIb/IIIa, purchased from Immunotech-Beckman-Coulter, Krefeld, Germany
- CD 42a: glycoprotein Ib, purchased from Dianova, Hamburg, Germany
- CD 61: fibrinogen receptor/glycoprotein IIIa, purchased from Becton Dickinson, Heidelberg, Germany
- CD 62P: detects P-selectin which is externalized upon degranulation of alpha granules, purchased from Dianova, Hamburg, Germany
- LIBS: ligand-induced binding site of the fibrinogen receptor, provided by Dr. Ginsberg, Scripps Institute, San Diego
- PAC-1: activated fibrinogen receptor, purchased from Becton Dickinson, Heidelberg, D
- CD 40-L: released upon dense granule degranulation; purchased from Immunotech-Beckman-Coulter, Krefeld, Germany

The antibodies were coupled to the following fluorescence systems, where appropriate (either directly or via secondary antibodies) and measured by fluorescence-activated cell sorting (FACS):
- FITC: fluorescein isothiocyanate, measured at 530 nm (green)
- PE: phycoerythrine, measured at 580 nm (red)
- PerCP: peridine chlorophyll protein, measured at 675 nm

Platelets which co-expressed the transgene GFP were gated for this marker by studying FITC fluorescence, and the same measurements were then exclusively related to this gate. The table shows that no significant differences in the activation (shift) of the various markers occurred between freshly isolated human platelets and stem cell-derived modified platelets which overexpressed GFP without further transgenes, although the basal absolute values differed somewhat (especially for CD 61).

| **Table of Results:** | | | | | | |
|---|---|---|---|---|---|---|
| | megakaryocyte-derived | | | freshly isolated | | |
| | basal | TRAP | ADP | basal | TRAP | ADP |
| CD 41 | 106±84 | 248±23 | 258±24 | 57±13 | 99±25 | 92±37 |
| CD 61 | 70±4,5 | 75±14 | 76±9,5 | 13±4 | 19±4.8 | 16±6 |
| CD42b | 49±16 | 75±41 | 46±25 | 36±23 | 40±2 | 58±15 |
| CD62P | 45±23 | 138±69 | 121±61 | 44±15 | 162±50 | 75±18 |
| PAC-1 | not defined | | | 26±11 | 122±34 | 90±26 |
| | | | | | | |
| LIBS | 97 | 157 (RGD) | | 9.7±6 | 49±35 (RGD) | |

| **Modified platelets expressing GFP:** | | | |
|---|---|---|---|
| | **basal** | **TRAP** | **ADP** |
| CD 41 | 179±33 | 213±34 | 216±18 |
| CD 61 | 303±75 | 325±73 | 333±78 |
| CD 42b | 73±25 | 81±29 | 95±63 |
| CD 62P | 166±55 | 299±146 | 314±153 |
| PAC-1 | 154±28 | 178±41 | 198±10 |

### Example 10: Nucleotide acid content in stem cell-derived platelets

The content of nucleic acids was compared in freshly isolated and ex vivo generated platelets by a fluorochrome staining assay. A stock solution of dye mix containing 100µg/mg acridine orange was prepared in PBS. Cells (1 x 10⁵) were suspended at in staining medium. Two microliters of dye mix were added to 50µl of cell suspension. Cells were stained for 20 minutes, then washed two times and analyzed by FACS at 530 nm (RNA) and 640 nm (DNA).

Fig. 4 shows that DNA extinction curves were very comparable in freshly isolated and in vitro generated platelets. For RNA extinction, somewhat higher values after in vitro culture were detected, corresponding to a higher amount of juvenile platelets in this sample.

### Example 11: Aggregation of platelets ex vivo

Washed platelets were resuspended in 50µL at 0.9 % NaCI. RED or RGD were added as required, where indicated, 10µl each, and incubated for 5 min. at RT. Fibrinogen was added to the CaCl₂ solution at a ratio of 5mL: 1.25mL CaCl₂ + 1.5mL. Fibrinogen (10 µg/µL) was used 20 µL /well. ADP was added in 10 µL. The mix was incubated at 37°C and photographed under normal and fluorescent light (480 nm). All steps were done in a reaction volume of 100 µL in 96 well-plate flate-bottom-plate.

The measurements (Fig. 5) showed that no aggregation occurred in the absence of calcium whereas there was a clear aggregation after addition of calcium and fibrinogen, which could be specifically blocked by the antagonist peptide RGD.

### Example 12: Release of active mediators from modified megakaryocytes and platelets

ELISA assays for β-thromboglobulin (Asserachrom β-TG, Roche Diagnostics, Mannheim, Germany) and platelet-derived growth factor (PDGF; PDGF-AB Immunoassay, Qiagen, Hilden, Germany) were performed according to the manufacturers' instructions. By using these assays, the thrombin-dependent release of both mediators from platelets was assessed.

Release of β-thromboglobulin from stem cell-derived platelets was 52±19 U/ml at basal conditions, 219±11 U/ml after addition of 0.2 U thrombin and 250±22 after 2 U thrombin (p<0.005 basal vs. thrombin). After infection with Ad-GFP, we detected a similar four-fold increase in βTG release. Also, in freshly isolated platelets, an increase from 59±6 to 115±10 U/ml occurred. The release of PDGF from stem cell-derived platelets was 280±25 pg/ml at basal conditions, 590±22 pg/ml after addition of 0.2 U thrombin, and 610 pg/ml after 2 U thrombin (p<0.05 basal vs. thrombin). After infection with Ad-GFP, an increase by 123±7 % was determined. In freshly isolated platelets, basal PDGF release (214±11 pg/ml) was similarly increased to 566±10 pg/ml by 0.2 U thrombin, and to 664±12 pg/ml by 1 U thrombin.

### Example 13: Labelling of platelets ex vivo and preparation of platelets for intravital microscopy

Modified platelets (50 x 10⁶) are resuspended in 0.25 ml of 38 mM citric acid/75 mM trisodium citrate/100 mM dextrose. Carboxyfluorescein diacetat succinimidyl ester (10ng/ml DCF, Molecular probes, USA) is added to label platelets *in vitro*. Alternatively, GFP-expressing platelets were used and studied for fluorescence microscopy without prior labelling, because they emitted at the wavelength used to detect FITC emission. After 5 min of incubation, the suspension is centrifuged repeatedly at 2000 × g for 10 min. The platelet pellet is resuspended in 0.25 ml phosphate-buffered saline (PBS, Seromed®, Berlin, Germany).

### Example 14: Intravital Microscopy after intestinal ischemia and reperfusion (I/R)

To investigate the role of potential platelet target proteins in diseases such as myocardial infarction, acute coronary syndrome or intestinal ischemia, modified platelets are investigated in intestinal ischemia and reperfusion injury in mice. To this end, fluorescent platelets are infused after intestinal I/R and visualized in the postischemic microcirculation by intravital fluorescence microscopy. Six-week-old inbred C57BL6/J mice are anaesthetized by inhalation of isoflurane-N₂O (0.35 FiO₂, 0.015 liter/liter isoflurane; Forene®; Abbott GmbH). The animals are placed on a heating pad (Effenberger), and polyethylene catheters (Portex) are implanted into the left carotid artery and left jugular vein for continuous recording of mean arterial blood pressure and infusion of fluorescent platelets, respectively. After laparotomy, a segment of the jejunum is exteriorized and constantly superfused with 37°C Ringer's lactat. Labeled, modified platelets (50 X 10⁶) are infused as bolus via the venous catheter into the acceptor mouse. Segmental jejunal ischemia is induced for 60 min by occluding the supplying vessels with microsurgical clips. Prior to induction of ischemia (baseline conditions) and after reperfusion, the intestinal segment is exposed on a mechanical stage and platelet-platelet (platelet aggregation) and platelet-endothelial cell interactions in the postischemic microvasculature are investigated by intravital microscopy. Prior to induction of ischemia and following the reperfusion, 10 non-overlapping regions of interest from the submucosal vessel of the ischemic/reperfused segment are randomly selected in each mouse and observed for 30 s with a modified microscope (Leitz). The microscopic images with a final magnification on the video screen of 450x are recorded by a CCD camera (FK6990, Cohu; Prospective Measurements) connected to a video recording system (Sony Corp.). Fluorescence is measured with adequate filters, e.g. with filters for FITC, GFP or rhodamine. For analysis of platelet-platelet and platelet-endothelial cell interactions, a computer-assisted image analysis program (CAP IMAGE; Dr. Zeintl, University of Heidelberg, Heidelberg, Germany) and frame-to-frame analysis of the videotapes are used. All experiments are performed in a blinded manner, and adherence of platelets to the surface of arterioles and venules (vessel diameter, 15-85 µm) and formation of platelet aggregates in capillaries (diameter, 15 µm), arterioles, and venules (vessel diameter, 15-85 µm) are quantified. The number of adherent platelets is assessed by counting the platelets that do not move or detach from the endothelial surface within 15 s. Platelet adhesion is presented per square millimeter of endothelial surface. The number of occluding and non-occluding aggregates is quantified within arterioles and venules and is presented per 100 vessels. To determine platelet aggregation in the capillary bed, the length (centimeter) of capillaries occluded by fluorescent platelets is measured and calculated per square centimeter of tissue cross-sectional area.

### Example 15: Preparation and Measurement of Platelet Adhesion in the Carotid Artery

To define the role of specific platelet target proteins for platelet adhesion to the vascular wall under physiological and pathophysiological conditions, such as atherosclerosis, modified platelets were also investigated in the carotid arteries of healthy or atherosclerotic mice. This approach allows to study their effects in cerebral vessels which play a pivotal role in the pathophysiology of stroke. Wild type C57BL6/J or atherosclerosis-prone ApoE-deficient mice are anesthetized by intraperitoneal injection of a solution of xylazine (5 mg/kg body weight, AnaSed; Lloyd Laboratories) and ketamine (80 mg/kg body weight, Ketaset; Aveco Co, Inc). Polyethylene catheters (Portex, Hythe, England) are implanted into the right jugular vein and fluorescent modified platelets (50×10⁶/250µl, see example 13) are infused intravenously. The right common carotid artery is dissected free for in situ assessment of platelet vessel wall interactions. Fluorescent platelets are visualized by *in vivo* videomicroscopy of the right common carotid bifurcation. We monitor platelet-endothelial cell interactions using a Zeiss Axiotech microscope (20 x water immersion objective, W 20x/0.5, Zeiss) with a 100W HBO mercury lamp for epi-illumination. All video-taped images are evaluated using a computer-assisted image analysis program (Cap Image 7.1, Dr. Zeintl, Heidelberg, Germany). Transiently adherent platelets are defined as cells crossing an imaginary perpendicular through the vessel at a velocity significantly lower than the centerline velocity; their numbers are given as cells per mm² endothelial surface. The number of adherent platelets is assessed by counting the cells that did not move or detach from the endothelial surface within 20 seconds. The number of platelet aggregates at the site of vascular injury was also quantified and is presented per mm².

### Example 16: Assessment of platelet adhesion and aggregation following vascular injury

To characterize the significance of specific platelet target proteins for platelet adhesion and aggregation on the subendothelial matrix following disruption of the endothelium, e.g. following balloon angioplasty or following spontaneous rupture of the atherosclerotic plaque, the adhesion and aggregation dynamics of transgene platelets are analyzed in a model of vascular injury of the mouse common carotid artery. Wild type C57BL6/J mice are anesthetized by intraperitoneal injection of a solution of xylazine (5 mg/kg body weight, AnaSed; Lloyd Laboratories) and ketamine (80 mg/kg body weight, Ketaset; Aveco Co, Inc). Polyethylene catheters (Portex, Hythe, England) were implanted into the right jugular vein and transgene platelets (50×10⁶/250µl) are infused intravenously. The right common carotid artery is dissected free and ligated vigorously near the carotid bifurcation for 5 min to induce vascular injury. This leads to complete loss of endothelial cells at the site of injury. Prior to and following vascular injury the fluorescent platelets are visualized in situ by in vivo video microscopy of the right common carotid bifurcation. Again, we monitor platelet-endothelial cell interactions using a Zeiss Axiotech microscope (20 x water immersion objective, W 20x/0.5, Zeiss) with a 100W HBO mercury lamp for epi-illumination. The definitions for transient and firm platelet adhesion were outlined above.

## Claims

1. A composition comprising platelets having a combination of the following modifications:
(a) a modification of the protein constituents of the platelets which is obtained or obtainable by genetic modification of platelet precursor cells;
(b) incorporation of a detectable label into said platelet;
whereby the functions of modification (a) and (b) are mutually independent.

2. The composition of claim 1, wherein modification (a) relates to
(i) the absence or inhibition of one or more proteins;
(ii) the overexpression of one or more proteins; and/or
(iii) the presence of a foreign protein capable of modifying an existing protein.

3. The composition according to claim 1 or 2, wherein modification (b) relates to the incorporation of a fluorescent dye or protein into the platelets.

4. Use of the composition of any one of the preceding claims as a probe for platelet function in a model animal or model animal blood vessel.

5. The use according to claim 4, wherein the model animal is a mouse, preferably a transgenic mouse such as an ApoE-deficient mouse.

6. The use according to any one of claims 4 to 5, wherein the platelet function involves aggregation and/or adhesion.

7. A method of determining platelet adhesion to a vessel wall of interest, whereby the method comprises the steps of
(a) exposing the interior wall of a blood vessel under predetermined conditions with a composition according to any one of claims 1 to 3;
(b) determining the adhesion of the detectably labelled platelets to the blood vessel wall.

8. The method according to claim 7, wherein the blood vessel is an ischemic blood vessel wall.

9. The method of claim 7, wherein the blood vessel an atherosclerotic blood vessel wall.

10. A method of determining platelet aggregation in a blood vessel of interest as compared to a reference vessel, whereby the method comprises the steps of
(a) exposing the interior wall of a blood vessel under predetermined conditions with a composition according to any one of claims 1 to 3;
(b) determining the aggregation of the detectably labelled platelets in the blood vessel.

11. The method according to claim 10, wherein the blood vessel is an ischemic blood vessel.

12. The method of claim 10, wherein the blood vessel an atherosclerotic blood vessel.

13. The method of any one of claims 7 to 12, wherein the vessels are portions of a carotid artery or an intestinal capillary bed.

14. The method of any of claims 7 to 13, wherein the label is detected by intravital videofluorescence microscopy.

15. A method for preparing platelets for a composition according to one of claims 1 to 3, wherein the method comprises the following steps:
(a) providing hematopoietic progenitor cells;
(b) generating megakaryocytes based on the hematopoietic progenitor cells of step (a);
(c) selecting a gene of interest from a gene library;
(d) transforming or transfecting the megakaryocytes with a vector containing the functional gene of interest of step (c) and optionally a gene coding for a detectable label;
(e) inducing shedding of platelets;
(f) isolating platelets containing expression products of the gene of interest and optionally a detectable label; and
(g) optionally incorporating a label into the platelets obtained in step (f).

16. The method according to claim 15, wherein a cytokine cocktail comprising thrombopoietin, interleukin-1β, stem cell factor and interleukin-6 is used in step (e).

17. A method for screening a gene library which comprises the method of any one of claims 4 to 14.

18. Method of validating a protein for determining its role in atherosclerosis or thrombosis for identifying a drug target wherein a composition according to any one of claims 1 to 3 is employed.

19. An assay for screening for drug candidates, wherein a drug target identified using a method according to one of claims 7 to 14 is used.

20. An assay for testing new drug candidates by using the composition of one of claims 1 to 3.
